# EUROPÄISCHE PATENTSCHRIFT

(11) **EP 3 366 123 B1**
(45) Veröffentlichungstag und Bekanntmachung des Hinweises auf die Patenterteilung: **20.05.2020**
(21) Anmeldenummer: 18000280.0
(22) Anmeldetag: 25.11.2016
(51) Int. Cl.: A01K 47/06, A61M 11/04, A61M 16/00, A61M 16/12, A61M 16/10, A61M 16/06

(54) **ANSAUGVORRICHTUNG ZUR NUTZUNG VON BIENENSTOCKLUFT**
INTAKE DEVICE FOR USE OF BEEHIVE AIR
DISPOSITIF D'ASPIRATION DESTINÉ À L'UTILISATION DE L'AIR DE RUCHE

(30) Priorität: 04.12.2015 DE 202015008332 U
(43) Veröffentlichungstag der Anmeldung: 29.08.2018
(62) Teilanmeldung aus: 16002541.7
(73) Patentinhaber: Schmiedgen, Jürgen, 09474 Crottendorf OT Waltersdorf (DE)
(72) Erfinder: Schmiedgen, Jürgen, 09474 Crottendorf OT Waltersdorf (DE)
(74) Vertreter: Kietzmann, Manfred

(56) Entgegenhaltungen:
- CH-A- 207 833
- DE-A1- 3 319 598
- DE-A1-102012 100 225
- DE-U1-202009 013 545
- DE-U1-202013 000 713
- US-A1- 2014 366 876
- US-A1- 2015 306 320

## Beschreibung

Die Erfindung betrifft eine Ansaugvorrichtung zur Nutzung von Bienenstockluft, nämlich zum Inhalieren derselben für den Menschen. Derartige Vorrichtungen sind bereits in den achtziger Jahren des letzten Jahrhunderts entwickelt worden, beispielsweise durch Heinrich Hüttner aus Österreich. Eine solche Vorrichtung ist beispielsweise in der DE 10 2012 100 225 A1 beschrieben. Eine ähnliche Vorrichtung geht ebenfalls aus der DE 20 2009 013 545 U1 CH 207 833 A und der DE 20 2013 000 731 U1 hervor.

Bekannte Vorrichtungen bestehen aus einer plattenartigen Ansaugvorrichtung aus Holz, welche in passenden Abmaßen plan auf eine Bienenstocköffnung eines Bienenstocks aufgesetzt ist und mit der Bienenstockluft angesaugt werden kann. Die Ansaugvorrichtung weist eine Auflageplatte zum horizontalen Aufsetzen auf den offenen Bienenstock mit einer Öffnung auf und ist so bienenstockseitig mit einer Einströmöffnung versehen. An diese Einströmöffnung schließt sich Bienenstock abgewandt ein Gebläse an, und zwar in Form eines von einem Gehäuse umgebenen handelsüblichen Axialventilators. Das Gebläse ist auf die Auflageplatte aufgesetzt. Die Einströmöffnung hat im Wesentlichen die Größe eines Ventilators des Gebläses, wie erwähnt in Form eines Axialventilators.

Im Wesentlichen zur Einströmöffnung gegenüberliegend ist an einer Ausströmöffnung der Ansaugvorrichtung eine Durchströmvorrichtung in Form eines Schlauches anschließbar, die in eine Abgabeeinrichtung mündet, welche zur Anlegung an eine Nase oder einen Mund eines Menschen vorgesehen ist, eine so genannte Atemmaske. Über diese atmet der Mensch im Rahmen der Anwendung der Vorrichtung Bienenstockluft ein und wieder aus. An der Einströmöffnung ist ferner ein Filter gelegen, der die Ansaugvorrichtung gegenüber dem Bienenstock abgrenzt, sodass sichergestellt ist, dass keine Bienen durch die Ansaugvorrichtung und so in die Durchströmeinrichtung gelangen können. Des Weiteren befindet sich bereits ein Gitter im Deckel des Bienenstockes. Durch Öffnen einer Abdeckung wird das Gitter freigelegt und die Auflageplatte kann aufgesetzt werden. Ferner ist das Gebläse regelbar.

Es hat sich herausgestellt, dass an der Ansaugvorrichtung aufgrund der Temperatur und Feuchtigkeitsunterschiede von Atemluft, Bienenstockluft und Umgebungsluft das Problem auftritt, dass sich innerhalb der vorbeschriebenen Vorrichtung Kondensat bildet. Die Kondensatbildung führt zu erheblichen Problemen in der Anwendungshygiene.

Bei einer bisherigen Holzkonstruktion kann es sogar zu Schimmelbildung kommen. Elektrische und elektronische Bauteile sind durch Kondensatbildung erheblich gefährdet. Es kommt - zu Zersetzungen von Lötstellen und Bauteilen. Dadurch kann die gesundheitliche Wirkung der Bienenstocklufttherapie beeinträchtigt werden.

Des Weiteren kommt es in Folge der Kondensatbildung zu Auswaschungen wertvoller Inhaltsstoffe der Bienenstockluft.

Die Aufgabe der Erfindung besteht darin, eine optimierte Vorrichtung zur Nutzung von Bienenstockluft bereitzustellen, so mit einer verbesserten Anwendungshygiene, bei welcher eine Kondensatbildung reduziert ist.

Die Aufgabe bzw. die Unteraufgaben werden durch eine Ansaugvorrichtung zur Nutzung von Bienenstockluft eines Bienenstocks mit den Merkmalen des Hauptanspruchs 1 gelöst, wobei die Unteransprüche weitere erfindungsgemäße Ausgestaltungsvarianten beinhalten.

Danach ist erfindungsgemäß vorgesehen, dass - unabhängig der Abmaße der Einströmöffnung - die erfindungsgemäße Ansaugvorrichtung ganz oder teilweise doppelwandig ausgestaltet ist. Dabei ist eine äußere Hülle der Ansaugvorrichtung ganz oder zumindest teilweise doppelwandig.

Die Ansaugvorrichtung zur Nutzung von Bienenstockluft eines Bienenstocks weist dabei eine Einströmöffnung und eine bienenstockabgewandte Ausströmöffnung auf. Die Ansaugvorrichtung ist auf eine Bienenstocköffnung des Bienenstocks aufsetzbar, wobei die Einströmöffnung bienenstockseitig angeordnet ist. An der Ausströmöffnung ist ein Gebläse angeordnet und deren Hülle ist ganz oder teilweise doppelwandig, wobei bei einer teilweisen Doppelwandigkeit entweder nur einzelne Elemente der Ansaugvorrichtung doppelwandig sind oder einzelne Elemente zumindest teilweise doppelwandig gestaltet sind. Wie bekannt, ist an der Ausströmöffnung oder an dem Gebläse eine Durchströmvorrichtung mit einer Ausgabevorrichtung oder nur eine Ausgabevorrichtung, wie bekannt, anschließbar.

Die Doppelwandigkeit gewährleistet eine thermische Isolation zwischen Bienenluft und Temperatur in einem Therapieraum und vermindert zumindest oder verhindert sogar die eingangs erläuterte Kondensatbildung mit den dargestellten nachteiligen Folgen. Ein in diesem Zusammenhang verwendeter Kunststoff ist bereits ein schlechter Wärmeleiter, würde aber nach einer Zeit auch einen Temperaturangleich zulassen, was zu einer Kondensatbildung führen würde.

Weist die Ansaugvorrichtung die Form einer Haube auf, so ist zumindest eine Mantelfläche der Haube doppelwandig ausgestaltet, sodass ein Großteil der Fläche der Ansaugvorrichtung doppelwandig ausgestaltet ist.

Wenn die Ansaugvorrichtung einen Ansaugtrichter aufweist, so ist vorgesehen, dass der Ansaugtrichter doppelwandig ist. Zumindest jedoch sollen Mantelflächen des Ansaugtrichters doppelwandig ausgestaltet sein.

In weiterer Ausgestaltung der Erfindung ist vorgesehen, dass ein durch die Doppelwandigkeit gebildeter Zwischenraum luftreduziert ist oder ein technisches Vakuum aufweist. Dadurch wird die thermisch isolierende Funktion verstärkt.

Als Ausgabevorrichtung kommt jedwede für die Luftaufnahme des Menschen geeignete Vorrichtung in Betracht, vorteilhafter Weise ein Mund- und/oder Nasenstück.

Die Ansaugvorrichtung weist allgemein eine Einströmöffnung und eine Ausströmöffnung auf. Die Ansaugvorrichtung ist dabei auf eine Bienenstocköffnung des Bienenstocks aufsetzbar. Im Wesentlichen erfolgt das Aufsetzen in bekannter Weise horizontal von oben auf den geöffneten Bienenstock. Die Einströmöffnung ist bienenstockseitig angeordnet. Sie entspricht aber im Wesentlichen ihren Abmaßen nach denen der Bienenstocköffnung, was bedeutet, dass die Einströmöffnung alle oder nahezu alle Wabengassen abdeckt. Ferner ist vorgesehen, dass die Ausströmöffnung eine um ein Mehrfaches geringere Querschnittsfläche aufweist, als die der Einströmöffnung. An der bienenstockabgewandten Ausströmöffnung ist ein Gebläse angeordnet.

Die Gestaltung der Einströmöffnung kann daher wie nachfolgend dargestellt erfolgen. Wenn die durch die Einströmöffnung geschaffene Ansaugfläche nicht die vorab dargestellten Abmaße aufweist, kommt es zu einer partiellen Ansaugung und Nachströmung der Luft über ein Flugloch des Bienenstocks, ohne dass die Luft sich genügend mit den Inhaltsstoffen im Bienenstock anreichern kann. Von der erfindungsgemäßen Ansaugvorrichtung werden alle oder nahezu alle Wabengassen erfasst, sodass die vorgenannten Nachteile nicht auftreten. Ferner ist so eine gleichmäßige Ansaugung der Bienenstockluft gewährt.

Die Ausströmöffnung weist, wie eingangs dargestellt, eine um ein Mehrfaches geringere Querschnittsfläche auf, als die der Einströmöffnung. Ihre Querschnittsfläche ist an die Vorgaben des Gebläses angepasst. Der Querschnitt beträgt in der Regel zwischen 20 bis 40 mm.

An der Ausströmöffnung oder an dem Gebläse selbst ist eine Durchströmvorrichtung mit einer Ausgabevorrichtung oder nur eine Ausgabevorrichtung anschließbar, sodass sichergestellt ist, dass ein Mensch die Bienenstockluft leicht inhalieren kann. So bildet die Ansaugvorrichtung mit der anschließbaren Durchströmvorrichtung eine funktionell zusammenwirkende Baugruppe. An der Ansaugvorrichtung können bei Bedarf aber auch beliebige in ihrer Funktion passende Durchströmvorrichtungen mit Ausgabevorrichtungen angeschlossen werden oder nur Ausgabevorrichtungen. Es ist jeweils nur sicherzustellen, dass eine Inhalation durch den Menschen gewährleistet ist, was grundsätzlich auch gewährleistet sein kann, wenn der Mensch im Bereich der Ausströmöffnung die Bienenstockluft einatmet.

Weiterhin kann vorgesehen sein, dass die anordenbare Durchströmvorrichtung variabel beheizbar ist, so ein beheizbarer variabler Schlauch, und die Durchströmvorrichtung im Betriebszustand eine regelbare Arbeitstemperatur von 35 bis 37 Grad aufweist, sodass es nicht mehr zu Kondensationserscheinungen kommt bzw. dies im Bereich der Durchströmvorrichtung minimiert ist.

Vorteilhafterweise ist die Ansaugvorrichtung eine sich zur Ausströmöffnung verjüngende ein- oder mehrstückige Haube, an deren Basis die Einströmöffnung gelegen ist.

Des Weiteren kann vorgesehen sein, dass die Ansaugvorrichtung aus einem sich zur Ausströmöffnung verjüngenden Ansaugtrichter und einem Gehäuse besteht, welcher das Gebläse mit einer Steuereinheit / Steuerung aufnimmt und lösbar mit dem Ansaugtrichter verbunden ist. Als Steuereinheit / Steuerung ist eine elektronische Steuerung vorgesehen, mit welcher sowohl die Drehzahl des Radialventilators regelbar ist, als auch die Temperatur der anordenbare Durchströmvorrichtung. Die Temperatur der anordenbaren Durchströmvorrichtung und/oder die Drehzahl des Gebläses und damit der Volumenstrom der Bienenstockluft können auch in Abhängigkeit von durch Sensoren ermittelten Eigenschaften der Bienenstockluft, wie den Vorbeschriebenen oder dem Kohlenmonoxidgehalt und/oder Luftfeuchtigkeit, geregelt werden und zwar im Rahmen einer bekannten automatischen Steuerung.

In einer weiteren möglichen Ausgestaltung ist das Gebläse, das im Bereich oder an der Ausströmöffnung angeordnet ist, reversibel befestigt. Die Reversibilität gewährleistet ein einfaches Auswechseln und Reinigen des Gebläses und des Ansaugtrichters sowie der weiteren Bauteile.

Die Ansaugvorrichtung ist stets in passenden Abmaßen auf einen Bienenstock aufsetzbar. Sie weist, wie vorbeschrieben, vorteilhafter Weise die Form einer Haube auf, wobei als Haube vielfältige Formen erfindungsgemäß in Betracht kommen, so Domartige oder Pyramidenförmige. Es ist lediglich eine in Bezug auf die Umgebungsbedingungen, wie Form und Lage des Bienenstocks strömungsgünstige Form zu wählen.

Vorteilhafterweise liegen Mittelpunkte der Querschnittsflächen der Einströmöffnung und der Ausströmöffnung auf einer gedachten Lotrechte, so einer Höhe h, wenn beispielsweise die Ansaugvorrichtung die Form eines Kreiskegelstumpfes oder auch eines Pyramidenstumpfes besitzt.

Die Haubenform ist aber nicht auf die vorgenannten Körper beschränkt. So sind auch andere Körperformen denkbar.

Erfindungsgemäß wird so eine optimierte Vorrichtung zur Nutzung von Bienenstockluft bereitgestellt. Bei dieser ist ferner die Anwendungshygiene verbessert, eine Kondensatbildung reduzierbar.

Die Erfindung wird nachfolgend anhand eines Ausführungsbeispiels unter Bezugnahme auf die Figuren weiter erläutert. Dabei ergeben sich weitere Vorteile, Merkmale und Ausgestaltungen der Erfindung.

Es zeigen:
**Fig. 1** eine schematische Darstellung der Ansaugvorrichtung in Schrägperspektive mit angeschlossener Durchströmvorrichtung mit und Ausgabevorrichtung,
**Fig. 2** eine schematische Darstellung der Haube der Ansaugvorrichtung.

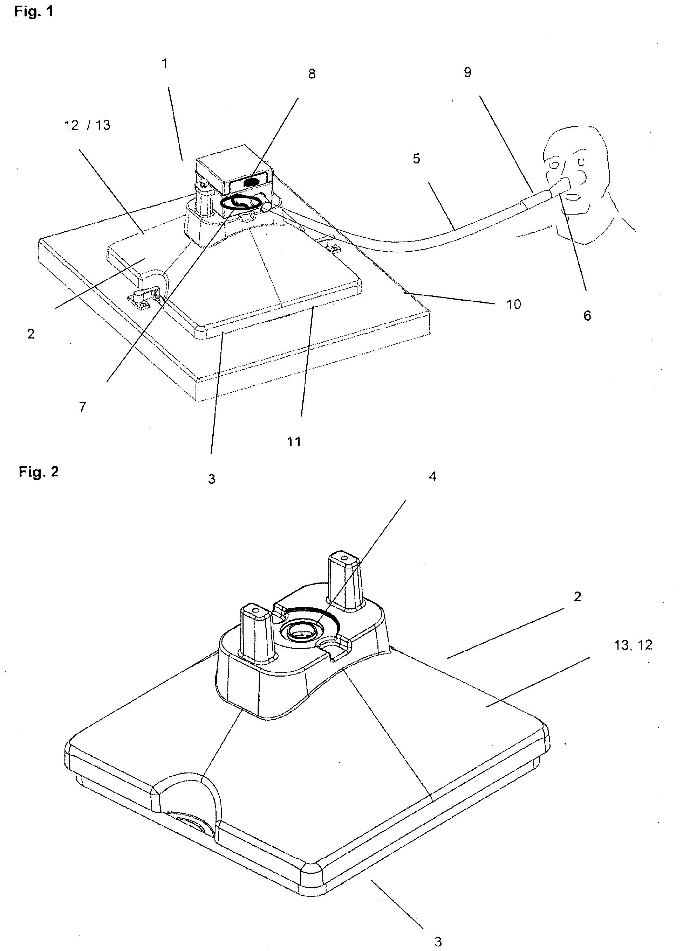

Danach weist die erfindungsgemäße Ansaugvorrichtung **1** eine Einströmöffnung **3** und eine Ausströmöffnung **4** auf. Im Ausführungsbeispiel ist die Ansaugvorrichtung eine sich zur Ausströmöffnung **4** verjüngende trichterförmige Haube **2,** an deren Basis die Einströmöffnung **3** gelegen ist. Die Ansaugvorrichtung **1** besitzt demgemäß einen Ansaugtrichter **12.** Die Mantelfläche **13** des Ansaugtrichters **12** ist doppelwandig. Die Materialstärke von jeweiligen Wänden beträgt ca. 2 mm, der Abstand zwischen einer Innen- und einer Außenwand beträgt ca. 16 mm, **Fig. 1, 2****.**

Die Ansaugvorrichtung **1** ist im Ausführungsbeispiel auf eine Bienenstocköffnung **11** eines Bienenstocks **10** aufgesetzt, **Fig. 1****.** Die bienenstockseitig angeordnete Einströmöffnung **3** entspricht ihren Abmaßen nach im Wesentlichen denen der Bienenstocköffnung **11** - sie überragt die Bienenstocköffnung geringfügig, um eine Auflage zu gewähren. Die Einströmöffnung **3** deckt so alle Wabengassen des Bienenstocks **10** ab.

An der bienenstockabgewandten Ausströmöffnung **4** ist ein Gebläse **7** in Form eines Radialventilators angeordnet, der in einem Gehäuse auf den Ansaugtrichter **13** aufgesteckt und reversibel arretiert ist. Im Gehäuse ist ferner eine Steuerung **8** angeordnet. Die Steuerung **8** dient zur manuellen Regulierung der Drehzahl des Radialventilators als Gebläse **7.** An dem Gebläse **7** ist eine Durchströmvorrichtung **5** mit einer Ausgabevorrichtung **6** anschließbar und zur besseren Darstellung angeordnet. Bei funktionsgerechter Anordnung der Durchströmvorrichtung **5** an das Gebläse **7** ist über die Steuerung **8** zudem die Temperatur der Durchströmvorrichtung **5** regelbar. Im Ausführungsbeispiel ist die Durchströmvorrichtung **5** ein beheizbarer Schlauch, an dem als Ausgabevorrichtung **6** ein übliches Nasen-/Mundstück für einen die Bienenstockluft inhalierenden Menschen angeschlossen ist.

Des Weiteren weist der Schlauch ein Ventil **9** auf, welches die aus dem Bienenstock **10** mittels des Gebläses 7 angesaugte Bienenstockluft nur in Richtung der Ausgabevorrichtung bzw. des die Bienenstockluft inhalierenden Menschen strömen lässt und bei einem Ausatmungsvorgang des inhalierenden Menschen schließt und sodann eine Membrane nach außen öffnet, sodass die verbrauchte Bienenstockluft an einen den Bienenstock umgebenden Raum abgegeben werden kann.

### Bezugszeichenliste:

- 1.: Ansaugvorrichtung
- 2.: Haube
- 3.: Einströmöffnung
- 4.: Ausströmöffnung
- 5.: Durchströmvorrichtung
- 6.: Ausgabevorrichtung
- 7.: Gebläse
- 8.: Steuerung
- 9.: Ventil
- 10.: Bienenstock
- 11.: Bienenstocköffnung
- 12.: Ansaugtrichter
- 13.: Mantelfläche

## Patentansprüche

1. Ansaugvorrichtung (1) zur Nutzung von Bienenstockluft eines Bienenstocks (10) zum Inhalieren derselben durch den Menschen mit einer Einströmöffnung (3) und einer bienenstockabgewandten Ausströmöffnung (4), wobei die Ansaugvorrichtung (1) auf eine Bienenstocköffnung (11) des Bienenstocks (10) aufsetzbar ist, die Einströmöffnung (3) bienenstockseitig angeordnet ist, an der Ausströmöffnung (4) ein Gebläse (7) angeordnet ist, und wobei eine äußere Hülle der Ansaugvorrichtung (1) ganz oder teilweise doppelwandig ist.

2. Ansaugvorrichtung (1) nach Anspruch 1, wobei die Ansaugvorrichtung (1) die Form einer Haube (2) besitzt und eine Mantelfläche (13) der Haube (2) doppelwandig ist.

3. Ansaugvorrichtung (1) nach Anspruch 1, wobei ein Ansaugtrichter (12) der Ansaugvorrichtung (1) oder seine Mantelfläche (13) doppelwandig ist.

4. Ansaugvorrichtung (1) nach einem der vorherigen Ansprüche 1 bis 3, wobei ein durch die Doppelwandigkeit gebildeter Zwischenraum luftreduziert ist oder ein technisches Vakuum aufweist.

## Claims

1. An intake device (1) for use of beehive air of a beehive (10) for inhaling the same by humans, having an inflow opening (3) and an outflow opening (4) facing away from the beehive, wherein the intake device (1) is placeable on a beehive opening (11) of the beehive (10), the inflow opening (3) is arranged on the side facing the beehive, a fan (7) is arranged at the outflow opening (4), and wherein an outer shell of the suction device (1) is either entirely or partially double-walled.

2. The intake device (1) according to Claim 1, wherein the suction device (1) has the shape of a hood (2) and an casing surface (13) of the hood (2) is double-walled.

3. The intake device (1) according to Claim 1, wherein an intake funnel (12) of the intake device (1) or the casing surface (13) of said funnel is double-walled.

4. The intake device (1) according to any one of the preceding Claims 1 to 3, wherein an interspace formed by the double wall is air-reduced or has a technical vacuum.

## Revendications

1. Dispositif d'aspiration (1) destiné à d'utilisation de l'air d'une ruche (10) afin de faire inhaler celui-ci par des humains, ayant une ouverture d'admission (3) et une ouverture d'évacuation (4) opposée à la ruche, dans lequel le dispositif d'aspiration (1) peut être placé sur une ouverture (11) de la ruche (10), l'ouverture d'admission (3) est disposée côté ruche, au niveau de l'ouverture d'évacuation (4) un ventilateur (7) est disposé, et dans lequel une enveloppe extérieure du dispositif d'aspiration (1) est partiellement ou totalement à double paroi.

2. Dispositif d'aspiration (1) selon la revendication 1, dans lequel le dispositif d'aspiration (1) a la forme d'une hotte (2) et une surface extérieure(13) de la hotte (2) est à double paroi.

3. Dispositif d'aspiration (1) selon la revendication 1, dans lequel un entonnoir d'aspiration (12) du dispositif d'aspiration (1) ou sa surface extérieure (13) est à double paroi.

4. Dispositif d'aspiration (1) selon une des revendications précédentes 1 à 3, dans lequel un espace intermédiaire formé par la double paroi est réduit en air ou présente un vide technique.
